# EUROPEAN PATENT SPECIFICATION

(11) **EP 3 307 183 B1**
(45) Date of publication and mention of the grant of the patent: **21.08.2024**
(21) Application number: 16812127.5
(22) Date of filing: 27.05.2016
(51) Int. Cl.: A61N 7/00

(54) **APPARATUS FOR DAMAGING OR DESTROYING ADIPOCYTES**
VORRICHTUNG ZUR SCHÄDIGUNG ODER ZERSTÖRUNG VON ADIPOZYTEN
APPAREIL POUR ENDOMMAGER OU DÉTRUIRE DES ADIPOCYTES

(30) Priority: 15.06.2015 US 201514739040
(43) Date of publication of application: 18.04.2018
(73) Proprietor: Mattioli Engineering Limited, London WC2E 9JT (GB)
(72) Inventor: BERNABEI, Gian Franco, 50121 Firenze (IT)
(74) Representative: Savi, Massimiliano
(86) International application number: PCT/US2016/034511
(87) International publication number: WO 2016/204957

(56) References cited:
- EP-A1- 2 586 395
- EP-A2- 2 252 369
- EP-B1- 2 252 369
- JP-A- S63 309 249
- US-A1- 2002 077 550
- US-A1- 2006 241 531
- US-A1- 2013 260 469
- US-B1- 6 368 281
- US-B2- 8 133 191
- US-B2- 8 579 835
- US-B2- 8 636 665

## Description

### FIELD OF THE INVENTION

This invention relates to an apparatus for applying transversal ultrasound waves to a patient's skin in order to damage and/or destroy adipocytes located under the dermis of the patient's skin.

### BACKGROUND

Procedures currently exist for removing fat cells under the skin, whereby those fat cells or adipose cells are also commonly referred to as "adipocytes." One such procedure ruptures adipocytes using longitudinal ultrasound waves, whereby ultrasound waves are applied to adipose tissue beneath the skin surface (the dermis). The ultrasound waves rupture the adipocytes in the adipose tissue under the skin surface, causing necrosis, which can cause extensive collateral damage to other non-fat tissue (e.g., blood vessels, connective tissue, dermis, etc.).

U.S. Patent No. 8,579,835 to Britva et al. describes an improved fat cell killing apparatus and method, which applies both transverse ultrasound waves and longitudinal ultrasound waves to a patient's skin. Britva uses a sonotrobe to apply the transverse ultrasound waves and longitudinal ultrasound waves to a patient's skin, whereby the sonotrobe has a curved distal portion having a plurality of undulations or ridges provided along the curved distal portion, for application of transverse ultrasound waves to a patient's skin.

Britva describes applying longitudinal ultrasound waves to a patient's skin during a hot mode of operation, and to apply transverse ultrasound waves to the patient's skin during a cold mode of operation, in order to enhance the destruction of adipocytes under the skin surface. Britva describes that adipocytes typically die within three days after treatment of a patient's skin with both transverse ultrasound waves and longitudinal ultrasound waves.

Britva describes the use of two resonant frequencies: a) a cold mode resonant frequency of about 69 kHz, and b) a hot mode resonant frequency of about 60 kHz. During operation in the cold mode, Britva's sonotrobe applies ultrasound vibrations in the distal portion of his sonotrobe primarily in a direction substantially perpendicular to the elongate neck axis (e.g., the longitudinal axis) of the sonotrobe, and whereby a transverse mechanical standing wave is generated in the distal portion of his sonotrobe by way of ridges that convert longitudinal waves to transverse waves, for application to the patient's skin. During operation in the hot mode, Britva's sonotrobe applies ultrasound vibrations in the distal portion of his sonotrobe primarily in a direction substantially parallel to the elongate neck axis of the sonotrobe, and whereby a longitudinal mechanical standing wave is generated in the distal portion of his sonotrobe.

Britva goes on to describe that application of ultrasound waves in the cold mode and the hot mode provides for good results regarding destruction of adipocytes under the skin surface.

Britva's ultrasound generator housed within a proximal part of his sonotrobe only outputs longitudinal waves, for which some of those waves are converted to transverse waves by way of the complex structure of his distal curved portion with plural ridges or undulations (as shown in Figures 9A-9C of Britva).

Further relevant background art is disclosed in documents JP S63309249 and EP 2586395.

### SUMMARY

According to the present invention there is provided an apparatus as claimed in claim 1. further embodiments of the anoaratus according to the present invention being defined by the dependent claims. Methods described hereinafter do not form part of the claimed invention.

One aspect of the subject matter described in this specification can be embodied in a apparatus for treating adipose tissue located beneath a patient's skin. The apparatus includes a main body portion that houses an ultrasound transducer, in which the main body portion is provided at a proximal end of the apparatus furthest from the patient's skin when the patient is being treated with the apparatus. The apparatus further includes a semisphere portion provided at a distal end of the apparatus and that is configured to contact the patient's skin when the patient is being treated with the apparatus. The apparatus further includes an intermediate portion provided between the main body portion and the semisphere portion, in which the intermediate portion is disposed substantially perpendicular to the main body portion such that a main axis of the main body portion is provided along a first plane substantially parallel to a second plane corresponding to a surface of the patient's skin being treated with the apparatus, and such that a main axis of the intermediate portion is provided along a third plane substantially perpendicular to the second plane. The ultrasound transducer is configured to vibrate along the first plane and to thereby cause the semisphere portion to vibrate substantially parallel to the patient's skin due to a connector provided within the intermediate portion that connects the ultrasound transducer to the semisphere. This results in transverse ultrasound waves being applied to the patient's skin by way of the apparatus, which results in destruction and/or damage to adipocytes located beneath a dermis of the patient's skin.

Another aspect of the subject matter described in this specification can be embodied in a method for treating adipose tissue located beneath a patient's skin. The method includes outputting transverse ultrasound vibrations from an ultrasound transducer provided in a proximal end of the probe to a first end of a connecting rod. The method also includes providing the transverse ultrasound vibrations from the first end of the connecting rod to a second end of the connecting rod that is connected to a semisphere portion located at a distal end of the probe. The transverse ultrasound vibrations are configured to be applied to the patient's skin by contacting the semisphere portion of the probe to the patient's skin.

### BRIEF DESCRIPTION OF THE DRAWINGS

The details of one or more embodiments of the subject matter described in this specification are set forth in the accompanying drawings and the description below. Other features, aspects, and advantages of the subject matter will become apparent from the description, the drawings, and the claims.
FIG. 1 shows a probe for applying transverse ultrasound waves to a patient's skin according to an embodiment;
FIG. 2 shows the separate elements of the probe of FIG. 1 in exploded view;
FIG. 3 shows one possible configuration of an ultrasound transducer that is provided in the probe of FIG. 1, according to an embodiment;
FIG. 4 shows the probe of FIG. 1 being applied to a patient's skin, in which transverse waves are applied to the patient's skin in order to destroy or damage adipose cells under the patient's skin, according to an embodiment;
FIG. 5 shows components housed within the probe of FIG. 1, which create transverse ultrasound vibrations and apply those vibrations to the patient's skin, according to an embodiment, and
FIGs. 6A and 6B show the semisphere portion of the probe of FIG. 1, in a front view and a side view, respectively.

Like reference numbers and designations in the various drawings indicate like elements.

### DETAILED DESCRIPTION

The present specification is directed to an apparatus and method for applying transversal ultrasound waves to a patient's skin in order to damage and/or destroy adipocytes located under the dermis of the patient's skin.

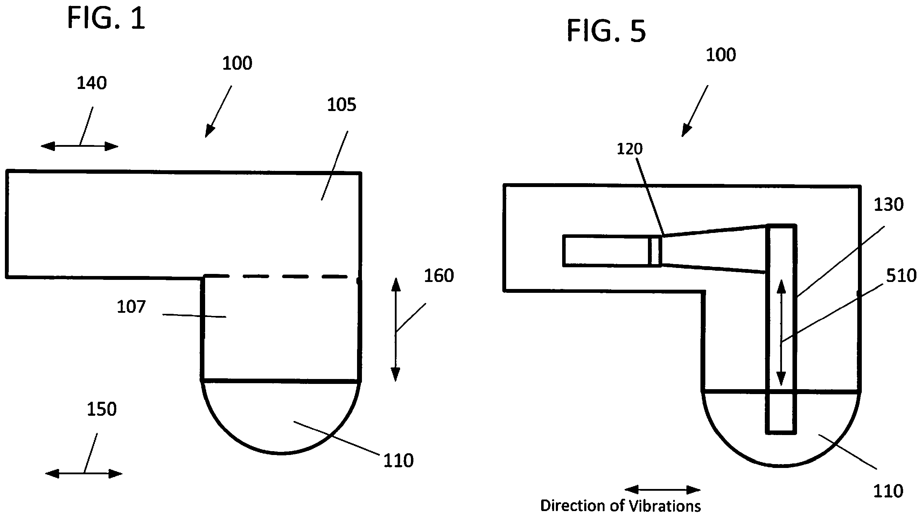

According to one or more embodiments, a probe provides transverse ultrasound vibrations to a patient's skin, whereby those transverse ultrasound vibrations impinge on the skin surface substantially parallel to the skin surface, and enter into the skin surface a predetermined depth, such as 20 - 40 mm before being substantially attenuated, so as to damage and/or destroy adipose cells within a certain range (e.g., 0.01 to 40 mm) under the skin surface. Fig. 1 shows a probe 100 according to one or more embodiments of the invention.

Referring now to Fig. 1 and Fig. 5, a distal end of the probe 100 includes a semisphere portion 110, whereby the semisphere portion 110 receives transverse ultrasound vibrations output from an ultrasound transducer 120, provided in a main body portion 105 of the probe 100 (that is provided at a proximal end of the probe 100). A metal component, also referred herein as a connector rod 130, is provided within an intermediate portion 107 of the probe 100, whereby the connector rod 130 transfers transverse ultrasound vibrations output from the ultrasound transducer 120 directly to the semisphere portion 110 of the probe, for application of those transverse ultrasound vibrations to the patient's skin.

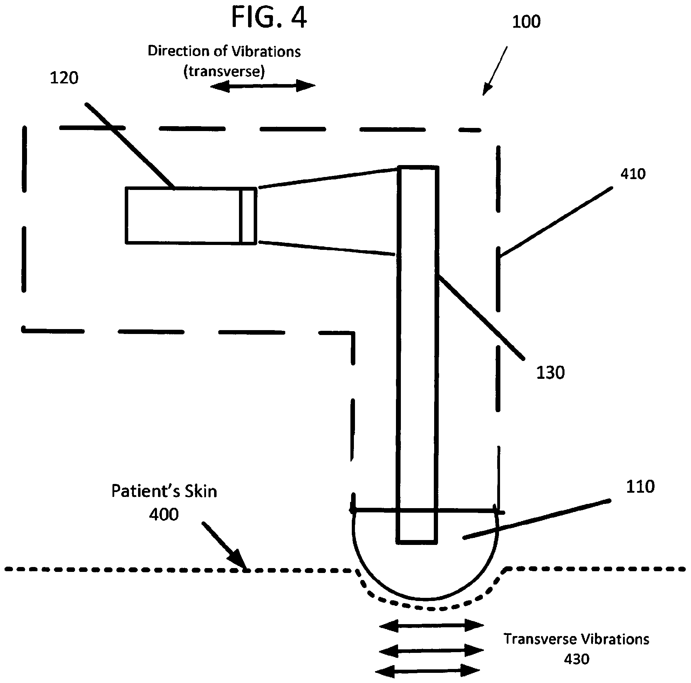

In one embodiment, the semisphere portion 110 of the probe 100 has a radius of 20 mm, so that transverse ultrasound vibrations are applied to a depth of 20 mm by way of pressing the semisphere portion of the probe 100 against the patient's skin during treatment of the patient's skin to damage and/or destroy adipose cells underneath the patient's skin. FIG. 4 shows the probe 100 according to an embodiment being pressed against the patient's skin 400, so that the semisphere portion 110 of the probe 100 is positioned 20 mm inward with respect to upper surfaces of the skin that are adjacent to but not in direct contact with the semisphere portion 110 of the probe 100. In FIG. 4, the housing of the probe 100 is shown by way of the dashed line region 410, whereby the components within the housing of the probe 100 that create the transverse ultrasound vibrations and provide those vibrations to the skin 400 are shown by way of the ultrasound transducer 120 and the connector rod 130. Also, the direction of transverse vibrations applied to be applied to the patient's skin 410 are shown by way of double-ended arrows 430.

In some embodiments, the strength of the transverse ultrasound vibrations is strong enough such that adipose cells located within a range up to 40 mm beneath the patient's skin are damaged and/or destroyed when subjected to those transverse ultrasound vibrations.

By providing an apparatus for directly applying transverse ultrasound waves output from an ultrasound transducer provided within a probe to a patient's skin, a less complex, easier-to-manufacture, and less-susceptible-to-malfunction probe than what is described in the Britva patent is obtained, and whereby the inventor of this application has determined that supplying only transverse ultrasound waves to a patient's skin provides a better effect than applying both transverse and longitudinal ultrasound waves at a same time period or at consecutive time periods.

Also, by utilizing a semisphere portion having a smooth outer surface at a distal end of the probe, whereby no ridges or undulations are provided on the outer surface of the semisphere portion (in contrast to the structure of Britva), a smoother treatment effect can be obtained, whereby the semisphere portion can easily glide over a portion of the patient's skin to be treated to damage and/or destroy adipose cells located beneath that portion of the patient's skin. Also, since there is no need to have a complex-shaped distal portion of the probe as required in Britva's structure to convert longitudinal vibrations to transverse vibrations, an easier-to-manufacture probe can be obtained, and as stated above, a more pleasant effect can be obtained during treatment of a patient's skin due to the smooth (e.g., non-undulating and non-ridged) shape of the semisphere portion of the probe that is in direct contact with the patient's skin.

The probe 100 of FIG. 1 includes a main body portion 105 that houses an ultrasound transducer 120 as seen in Fig. 5, in which the main body portion 105 is provided at a proximal end of the probe 100 furthest from the patient's skin when the patient is being treated with the probe 100. The probe 100 further includes a semisphere portion 110 provided at a distal end of the probe 100 that is configured to contact the patient's skin when the patient is being treated with the probe 100. The probe 100 further includes an intermediate portion 107 provided between the main body portion 105 and the semisphere portion 110, in which the intermediate portion 107 is disposed substantially perpendicular to the main body portion 105 such that a main axis of the main body portion 105 is provided along a first plane 140 substantially parallel to a second plane 150 corresponding to a surface of the patient's skin being treated with the probe 100, and such that a main axis of the intermediate portion 107 is provided along a third plane 160 substantially perpendicular to the second plane.

The ultrasound transducer is configured to vibrate along the first plane 140 and to thereby cause the semisphere portion 110 to vibrate substantially parallel to the patient's skin due to a connector provided within the intermediate portion that connects the ultrasound transducer to the semisphere. This results in transverse ultrasound waves being applied to the patient's skin by way of the probe 100, which results in destruction and/or damage to adipocytes located beneath a dermis of the patient's skin.

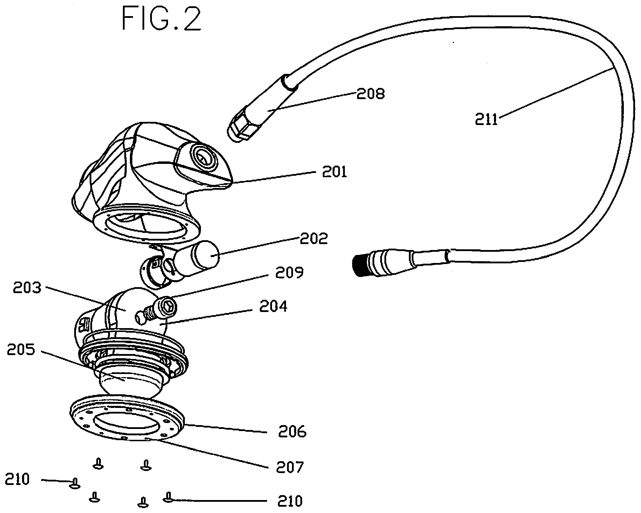

FIG. 2 shows in exploded view various components that may be utilized to create a probe 100 according to one or more embodiments. Those components include:
- 201.: Upper plastic housing
- 202.: (optional) electric motor with eccentric in order to generate additional acoustic waves, such as between 50- 100 Hz as per the dermoelectroporation technology
- 203.: Ultrasound Transducer
- 204.: Connector rod
- 205.: Semisphere portion attached to the connector rod
- 206.: Lower plastic housing
- 207.: (optional) metal electrodes used to apply additional electric current to the patient's skin, according to the dermoelectroporation technology.
- 208.: Electrical cables inlet housing
- 209.: Screw of the connection rod
- 210.: Screws (6 shown in Fig. 2) for attaching the lower plastic housing to the probe
- 211.: Electrical cable connected to the inlet housing

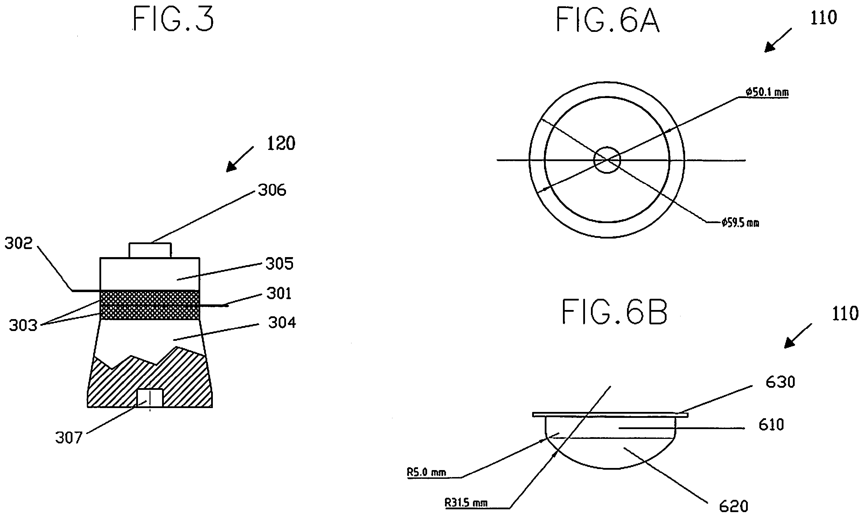

FIG. 3 shows details of an ultrasound transducer 120 that can be provided within the main body portion 105 of the probe 100, according to one or more embodiments. The ultrasound transducer 120 may be constructed as a piezoelectric device or other type of device that provides ultrasound vibrations. In some configurations, two piezoelectric plates are coupled to each other, with one plate causing a vibration in an opposite direction with respect to the other plate. According to one embodiment, the ultrasound transducer (having a total length of 57 mm, a minimum width of 38 mm, and a maximum width of 48 mm) includes:
- 301.: First piezoelectric crystal connection
- 302.: Second piezoelectric crystal connection
- 303.: Piezoelectric crystal
- 304.: Front metal part (having a maximum width of 48 mm)
- 305.: Back metal part (having a width of 38 mm, and a length of 23.5 mm)
- 306.: Screw for keeping all parts connected to each other (a top part of the screw extends out 5.5 mm from the back metal part)
- 307.: Hole for receiving the screw of the connector rod (7 mm long by 10 mm wide)

With reference to Fig. 1 and Fig. 5, the ultrasound transducer 120 is housed in the main body portion 105 of the probe 100, which has a main axis 140 that is substantially parallel to the patient's skin to be treated by the probe. With such a construction, the ultrasound transducer 120 outputs vibrations that are substantially parallel to the patient's skin, and thus are transverse ultrasound vibrations.

The transverse ultrasound vibrations output from the ultrasound transducer are transferred to the semisphere portion of the probe by way of a metal plate, or connector rod 130, as shown in FIG. 5. The connector rod 130 may be configured as having one main axis 510, and thus corresponding to a long rod-shaped structure. The connector rod 130 may be of an aluminum construction (e.g., an aluminum plate) or of another lightweight metal construction. One end of the connector rod 130 is in direct contact to the ultrasound transducer 120 and directly receives the ultrasound transverse vibrations output from the ultrasound transducer 120. The other end of the connector rod 130 is in direct contact with the semisphere portion 110 of the probe 100, and thus transfers the ultrasound transverse vibrations output from the ultrasound transducer 120 directly to the semisphere portion 110 of the probe 100, and thus directly to the patient's skin in contact with the semisphere portion 110 of the probe 100. With reference to Fig. 1 and FIG. 5, the connector rod 130 is housed primarily in the main body portion 105 and the intermediate portion 107 of the probe, whereby a proximal end of the connector rod is housed within the main body portion of the probe 100 and whereby a distal end of the connector rod 130 is housed within the semisphere portion 110 of the probe 100.

In some embodiments, to maintain as lightweight a construction as possible, the connector rod 130 has many holes provided along its main axis, as does the semisphere portion 110 of the probe 100. Also, due to the fact that the speed of ultrasound vibrations traveling on metal, such as aluminum, is about five (5) times the speed of ultrasound waves traveling on a patient's skin, the metal connector rod 130 can be considered to be rigid as compared to the patient's skin. This is also the case with respect to the semisphere portion 110 of the probe 100 that is connected to the connector rod 130, which can also be considered to be rigid with respect to the patient's skin. Due to the holes provided along the connector rod 130 and along the outer surface of the semisphere portion 110, the connector rod/semisphere structure has a mass weight less than the mass weight of the skin that it is to drive with transverse ultrasound vibrations. This provides an optimal way to apply transverse ultrasound vibrations to the patient's skin, so as to achieve a good effect for damaging and/or destroying adipose cells under the patient's skin (e.g., between 2 to 40 mm under the dermis of the skin). The holes provided on the outer surface of the semisphere portion 110 may be in the range of from 0.01 to 0.1 mm, so that they do not cause any discomfort when the semisphere portion 110 is slid over a portion of the patient's skin to be treated by way of the probe 100.

Due to the semisphere portion 110 having a radius of 20 mm in some embodiments, the first 20 mm under the patient's skin are subject to the transverse ultrasound vibrations as the semisphere portion 110 of the probe 100 is pressed against the patient's skin 400, as shown in FIG. 4. These transverse ultrasound vibrations have a maximum intensity at around 20 mm under the skin surface (for a semisphere portion 110 having a 20 mm radius), which is determined by the inventor to be an optimal depth for damaging and destroying adipocytes under the skin surface.

In other embodiments, the semisphere portion 110 of the probe 100 has a different size, such as between 15 mm to 25 mm, whereby similar positive effects by damaging and destroying adipocytes under the skin surface are obtained for such structures.

FIGs. 6A and 6B respectively show a front view and a side view of the semisphere portion of the probe, according to one or more embodiments. By way of example and not by way of limitation, the semisphere portion 110 includes a flat-sided base portion 610 that is of 8 mm in depth, and a curved portion 620 that is of 12 mm in depth with respect to a point of the curved portion 620 farthest from the base portion 610. By way of example and not by way of limitation, the semisphere portion110 has a thin plate portion 630 of 1.5 mm, for attachment to the intermediate portion 107 of the probe (see FIGs. 1 and 5, for example). The attachment of the thin plate portion 630 (and thus the semisphere portion 110) to the rest of the probe 100 may be by way of screws or other fixation devices (see screws 210 in Fig. 2, for example). In the embodiment as shown in FIG. 6A, the thin plate portion 630 is of a circular shape and has a diameter of 59.5 mm, and the flat-sided base portion 610 and the curved portion 620 of the semisphere 110 have a diameter of 50.1 mm. According to one embodiment, the curvature radius of the semisphere portion 110 is 31.52 mm, and the curvature radius of the part that connects the semisphere portion 110 to the 8 mm (in length) cylinder is 5 mm. Other curvature radiuses can be utilized.

By having a smooth shaped semisphere portion 110 of the probe 100 that is in direct contact with a skin surface to the treated, a good massaging effect can be obtained to the patient at the same time adipose cells are damaged and destroyed beneath the patient's skin. This dual benefit provides for a pleasant treatment experience for removing fat cells underneath a patient's skin. In some embodiments, an oil-based gel or other type of lubricating gel may be applied to the semisphere portion outer surface, to enhance the massaging effect when the semisphere portion is slid across the patient's skin. For example, in some embodiments, a gel-holding region within the semisphere portion 110 of the probe 100 may be included in some embodiments, whereby gel is output from the gel-holding region and through holes on the exterior housing of the semisphere portion 110 of the probe 100, and thereby onto the patient's skin, to enhance the movement of the semisphere portion 110 of the probe on the patient's skin during treatment of the patient. Actuation of a trigger (not shown in the drawings) on the probe 100 by a user of the probe 100 causes expelling of the gel from the gel-holding region, through the holes of the semisphere portion 110 of the probe 100, and thereby onto the patient's skin.

In some embodiments, the probe 100 has its own power supply (not shown in the drawings), such as a battery pack, and in other embodiments, the probe is configured to have an electrical cord that can be connected to an electrical output, to provide the necessary power to the components within the probe 100.

In some embodiments, the transverse ultrasound vibrations are provided in pulses of energy to the patient's skin, such as at a 20% duty cycle. Thus, for an output power of 20 - 35 watts/cm² output by the ultrasound transducer, the average power applied to the patient's skin at a 20 - 50% duty cycle is about 1 - 7 watts/cm², thereby providing a power flux to the patient's skin of 1 - 7 watts/cm², which does not cause much if any discomfort to the patient during treatment of the patient's skin.

In some embodiments, the ultrasound frequency of the transverse ultrasound vibrations is 32 kHz, and in other embodiments the ultrasound frequency of the transverse ultrasound vibrations is a frequency in the range of from 28 - 60 kHz.

Thus, particular embodiments of the subject matter have been described. In addition, the processes depicted in the accompanying figures do not necessarily require the particular order shown, or sequential order, to achieve desirable results.

## Claims

1. An apparatus (100) for treating adipose tissue located beneath a skin of a patient, comprising:
a main body (105) portion that houses an ultrasound transducer (120),
a semisphere portion (110) provided at distal end of the apparatus and configured to be pressed against the skin to bend the skin, wherein the semisphere portion (110) has a smooth outer surface without any ridges and undulations and is configured to be moved on the skin while the semisphere portion (110) is pressed, and
an intermediate portion (107) provided between the main body portion (105) and the semisphere portion (110), wherein the intermediate portion (170) further comprises a connector rod (130) provided between the ultrasound transducer (120) and the semisphere portion (110) and configured to transfer ultrasound transverse vibrations from the ultrasound transducer (120) to the semisphere portion (110) causing the semisphere portion (110) to vibrate in the same direction of the ultrasound transducer (120), the direction of vibration being parallel to the skin of the patient, thus generating at a predetermined depth transverse waves in direction parallel to the skin; **characterized in that** said transducer (120) is positioned in such a way to vibrate in a direction parallel to the skin of the patient.

2. The apparatus according to claim 1, wherein the transverse ultrasound vibrations are within the range of 28 - 60 KHz.

3. The apparatus according to claim 2, wherein the transverse ultrasound vibrations are pulsed at a duty cycle between 20 % and 50 %.

4. The apparatus of claim 1, wherein the semisphere portion (110) is a segment of a sphere and is configured to bend the skin inward thus generating the transverse waves at a depth between 0.1mm - 40mm under the skin.

5. The apparatus according to claim 2, wherein a power flux applied to patient skin is in the range between 1 - 3 watts/cm2.

## Patentansprüche

1. Vorrichtung (100) zur Behandlung von Fettgewebe, das sich unter einer Haut eines Patienten befindet, umfassend:
einen Hauptkörperabschnitt (105), der einen Ultraschallwandler (120) aufnimmt,
einen Halbkugelabschnitt (110), der am distalen Ende der Vorrichtung bereitgestellt ist und konfiguriert ist, um gegen die Haut gedrückt zu werden,
um die Haut zu biegen, wobei der Halbkugelabschnitt (110) eine glatte Au-ßenfläche ohne Rippen und Wellungen aufweist und konfiguriert ist, um auf der Haut bewegt zu werden, während der Halbkugelabschnitt (110) gedrückt wird, und
einen Zwischenabschnitt (107), der zwischen dem Hauptkörperabschnitt (105) und dem Halbkugelabschnitt (110) bereitgestellt ist, wobei der Zwischenabschnitt (170) ferner eine Verbindungsstange (130) umfasst, die zwischen dem Ultraschallwandler (120) und dem Halbkugelabschnitt (110) bereitgestellt ist und konfiguriert ist, um transversale Ultraschallschwingungen vom Ultraschallwandler (120) auf den Halbkugelabschnitt (110) zu übertragen, was bewirkt, dass der Halbkugelabschnitt (110) in derselben Richtung des Ultraschallwandlers (120) schwingt, wobei die Schwingungsrichtung parallel zur Haut des Patienten ist, wodurch in einer vorbestimmten Tiefe transversale Wellen in Richtung parallel zur Haut erzeugt werden, **dadurch gekennzeichnet, dass** der Wandler (120) so positioniert ist, dass er in einer Richtung parallel zur Haut des Patienten schwingt.

2. Vorrichtung nach Anspruch 1, wobei die transversalen Ultraschallschwingungen im Bereich von 28 - 60 KHz liegen.

3. Vorrichtung nach Anspruch 2, wobei die transversalen Ultraschallschwingungen mit einem Tastverhältnis zwischen 20 % und 50 % gepulst sind.

4. Vorrichtung nach Anspruch 1, wobei der Halbkugelabschnitt (110) ein Segment einer Kugel ist und konfiguriert ist, um die Haut nach innen zu biegen, wodurch die transversalen Wellen in einer Tiefe zwischen 0,1 mm - 40 mm unter der Haut erzeugt werden.

5. Vorrichtung nach Anspruch 2, wobei ein Leistungsfluss, der auf die Haut des Patienten angewendet wird, im Bereich zwischen 1 - 3 Watt/cm2 liegt.

## Revendications

1. Appareil (100) pour traiter le tissu adipeux situé sous la peau d'un patient, comprenant :
une partie de corps principal (105) qui abrite un transducteur ultrasonore (120),
une partie hémisphérique (110) prévue à l'extrémité distale de l'appareil et configurée pour être pressée contre la peau pour plier la peau, dans lequel la partie hémisphérique (110) a une surface extérieure lisse sans crêtes ni ondulations et est configurée pour être déplacée sur la peau pendant que la partie hémisphérique (110) est pressée, et
une partie intermédiaire (107) prévue entre la partie de corps principal (105) et la partie hémisphérique (110), la partie intermédiaire (170) comprenant en outre une tige de raccordement (130) prévue entre le transducteur ultrasonore (120) et la partie hémisphérique (110) et configurée pour transférer les vibrations transversales ultrasonores du transducteur ultrasonore (120) à la partie hémisphérique (110) provoquant la vibration de la partie hémisphérique (110) dans la même direction que le transducteur ultrasonore (120), la direction de vibration étant parallèle à la peau du patient, générant ainsi à une profondeur prédéterminée des ondes transversales dans une direction parallèle à la peau ; **caractérisé en ce que** ledit transducteur (120) est positionné de manière à vibrer dans une direction parallèle à la peau du patient.

2. Appareil selon la revendication 1, dans lequel les vibrations ultrasonores transversales sont dans la plage de 28 à 60 KHz.

3. Appareil selon la revendication 2, dans lequel les vibrations ultrasonores transversales sont pulsées à un cycle de service entre le 20 % et le 50 %.

4. Appareil selon la revendication 1, dans lequel la partie hémisphérique (110) est un segment de sphère et configurée pour plier la peau vers l'intérieur, générant ainsi les ondes transversales à une profondeur entre 0,1 mm et 40 mm sous la peau.

5. Appareil selon la revendication 2, dans lequel un flux de puissance appliqué à la peau du patient est dans la plage de 1 à 3 watts/cm2.
